(19) Europäisches Patentamt
European Patent Office
Office européen des brevets

(11) Veröffentlichungsnummer: **0 069 696**
**B1**

(12) # EUROPÄISCHE PATENTSCHRIFT

(45) Veröffentlichungstag der Patentschrift: **15.01.86**

(51) Int. Cl.⁴: **C 07 C 177/00**, C 07 C 35/48, A 61 K 31/557

(21) Anmeldenummer: **82730087.2**

(22) Anmeldetag: **25.06.82**

(54) **9-Fluor-prostaglandinderivate, Verfahren zur Herstellung und Verwendung als Arzneimittel.**

(30) Priorität: **03.07.81 DE 3126924**

(43) Veröffentlichungstag der Anmeldung: **12.01.83 Patentblatt 83/2**

(45) Bekanntmachung des Hinweises auf die Patenterteilung: **15.01.86 Patentblatt 86/3**

(84) Benannte Vertragsstaaten: **AT BE CH DE FR GB IT LI LU NL SE**

(56) Entgegenhaltungen:
**EP-A-0 030 377**
**DE-A-2 628 364**

**PROSTAGLANDINS, Band 16, Nr. 1, Juli 1978, Seiten 47-65, C.E. ARRONIZ et al.: "Synthesis of ring halogenated prostaglandins (1)"**
**CHEMISTRY, BIOCHEMISTRY & PHARMACOLOGICAL ACTIVITY OF PROSTANOIDS, 1978, Seiten 39-60, Pergamon Press, J.M. MUCHOWSKI: "Synthesis and bronchial dilator activity of some novel prostaglandin analogues"**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178** ***Postfach 65 03 11, D-1000 Berlin 65 (DE)***

(72) Erfinder: **Skuballa, Werner, Dr., Olwenstrasse 23, D-1000 Berlin 28 (DE)**
Erfinder: **Radüchel, Bernd, Dr., Gollanczstrasse 132, D-1000 Berlin 28 (DE)**
Erfinder: **Schwarz, Norbert, Dr., Stubenrauchstrasse 31, D-1000 Berlin 41 (DE)**
Erfinder: **Vorbrüggen, Helmut, Prof., Wilkestrasse 7, D-1000 Berlin 27 (DE)**
Erfinder: **Elger, Walter, Dr., Schorlemer Allee 12b, D-1000 Berlin 33 (DE)**
Erfinder: **Loge, Olaf, Dr., Bekassinen Weg 37, D-1000 Berlin 27 (DE)**
Erfinder: **Town, Michael- Harold, Dr., Buchsbaumweg 3, D-1000 Berlin 47 (DE)**

**EP 0 069 696 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft neue 9-Fluor-prostaglandinderivate, Verfahren zu ihrer Herstellung sowie ihre Verwendung als Arzneimittel.

Aus dem sehr umfangreichen Stand der Technik der Prostaglandine und ihrer Analoga weiss man, dass diese Stoffklasse aufgrund ihrer biologischen und pharmakologischen Eigenschaften zur Behandlung von Säugetieren, einschliesslich des Menschen, geeignet ist. Ihre Verwendung als Arzneimittel stösst jedoch häufig auf Schwierigkeiten. Die meisten natürlichen Prostaglandine besitzen eine für therapeutische Zwecke zu kurze Wirkungsdauer, da sie zu rasch durch verschiedene enzymatische Prozesse metabolisch abgebaut werden. Alle Strukturveränderungen haben das Ziel, die Wirkungsdauer sowie die Selektivität der Wirksamkeit zu steigern.

9-Desoxy-9-halogen-prostaglandinderivate sind bekannt. So beschreibt z.B. die EP-A-0030377 9-Chlor-Derivate.

Durch die deutsche Offenlegungsschrift 26 28 364 sowie die Publikationen von C.E. Arroniz et al. in Prostaglandins 16, 47(1978) und J.M. Muchowski in Chemistry, Biochemistry and Pharmacological Activity of Prostanoids (Pergamon Press 1978, 39) sind auch Prostaglaninderivate mit einem Fluoratom in 9-stellung bekannt.

Die in dieser Offenlegungsschrift beanspruchten Verbindungen enthalten als einzige Strukturvariante zu den natürlichen Seitenketten der Prostaglandine eine Alkylgruppe in der 15-Position.

Es wurde nun gefunden, dass durch weitere Strukturveränderungen in der unteren Seitenkette und/oder in der 1-Position der 9-Fluor-Prostaglandine eine längere Wirkungsdauer, grössere Selektivität und bessere Wirksamkeit erzielt werden kann. Die Erfindung betrifft 9-Fluor-prostaglandinderivate der allgemeinen Formel I

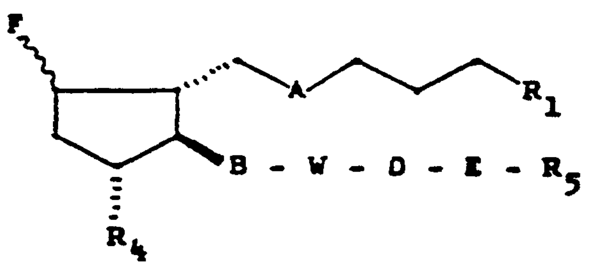

(I) ,

worin $R_1$ den Rest $CH_2OH$ oder

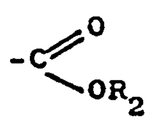

mit $R_2$ in der Bedeutung eines Wasserstoffatoms oder einer Alkylgruppe mit 1-4 C-Atomen, oder $R_1$ den Rest

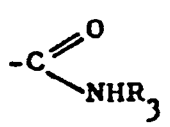

mit $R_3$ in der Bedeutung eines $C_{1-10}$-Alkanoyl- oder Methan- oder Isopropansulfonylrestes oder des Restes $R_2$ und A eine $-CH_2-CH_2-$ oder cis-$CH=CH$-Gruppe,

B eine $-CH_2-CH_2-$ oder trans-$CH=CH$- oder eine $-C\equiv C$-Gruppe,

W eine freie oder funktionell abgewandelte Hydroxymethylengruppe, wobei die OH-Gruppe α- oder β-ständig sein kann,

D und E gemeinsam eine direkte Bindung oder

D eine gerad- oder verzweigtkettige Alkylengruppe mit 1-10 C-Atomen, die gegebenenfalls durch Fluoratome substituiert sein kann,

E ein Sauerstoff- oder Schwefelatom, eine direkte Bindung, eine $-C\equiv C$-Gruppe oder eine $-CR_6=CR_7$-Gruppe darstellt, wobei $R_6$ und $R_7$ sich unterscheiden und ein Wasserstoffatom, ein Chloratom oder eine Alkylgruppe

bedeuten,

$R_4$ eine freie oder funktionell abgewandelte Hydroxygruppe,

$R_5$ ein Wasserstoffatom, eine gerad- oder verzweigtkettige, gesättigte oder ungesättigte Alkylgruppe mit 1-6 C-Atomen oder, wenn E ein Sauerstoffatom bedeutet, eine Phenylgruppe beteuten, wobei die Gruppierung -DER$_5$ nicht den Rest n-Pentyl darstellt,

und falls $R_2$ die Bedeutung eines Wasserstoffatoms hat, deren Salze mit physiologisch verträglichen Basen.

Das Fluoratom in 9-Position der Formel I kann sowohl α- als auch β-ständig sein.

Als Alkylgruppen $R_2$ sind gerade oder verzweigte Alkylgruppen mit 1-4 C-Atomen zu betrachten, wie beispielsweise Methyl, Äthyl, Propyl, Butyl, Isobutyl, tert.-Butyl.

Als Säurerest $R_3$ kommen physiologisch verträgliche Säurereste in Frage. Bevorzugte Säuren sind organische Carbonsäuren mit 1-10 Kohlenstoffatomen, die der aliphatischen, cycloaliphatischen, aromatisch-aliphatischen und heterocyclischen Reihe angehören sowie Sulfonsäuren. Diese Carbonsäuren können gesättigt, ungesättigt und/oder mehrbasisch sein. Beispielsweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Trimethylessigsäure, Diäthylessigsaure, tert.-Butylessigsäure, Cyclopropylessigsäure, Cyclopentylessigsäure, Cyclohexylessigsäure, Cyclopropancarbonsäure, Cyclohexancarbonsäure, Phenylessgisäure, Adipinsäure, Benzoesäure, Nikotinsäure, Isonikotinsäure, Furan-2-carbonsäure, Cyclopentylpropionsäure.

Als Sulfonsäuren kommen beispielsweise Methansulfonsäure, Äthansulfonsäure und Isopropansulfonsäure in Frage.

Die Hydroxygruppen in W und $R_4$ können funktionell abgewandelt sein, beispielsweise durch Verätherung oder Veresterung, wobei auch die abgewandelte Hydroxygruppe in W α- oder β-ständig sein kann.

Als Äther- und Acylreste kommen die dem Fachmann bekannten Reste in Betracht. Bevorzugt sind leicht abspaltbare Ätherreste, wie beispielsweise der Tetrahydropyranyl-, Tetrahydrofuranyl-, α-Athoxyäthyl-, Trimethylsilyl-, Dimethylsilyl-, tert.-Butyl-silyl- und Tribenzyl-silylrest. Als Acylreste kommen die gleichen wie für $R_3$ genannt in Frage, namentlich genannt seien beispielsweise Acetyl, Propionyl, Butyryl und Benzoyl.

Als Alkylgruppen $R_5$ kommen gerad- und verzweigtkettige, gesättigte und ungesättigte Alkylreste mit 1-6 C-Atomen in Frage. Beispielsweise genannt seien Methyl-, Äthyl-, Propyl-, Butyl-, Isobutyl-, tert.-Butyl-, Pentyl-, Hexyl-, Butenyl-, Isobutenyl-, Propenyl-, Pentenylgruppen.

Wenn E ein Sauerstoffatom bedeutet, kann $R_5$ auch für eine Phenylgruppe stehen.

Als Alkylengruppe D kommen geradkettige oder verzweigtkettige, gesättigte und ungesättigte Alkylenreste, vorzugsweise gesättigte mit 1-10, insbesondere 1-5 C-Atomen, in Frage, die gegebenenfalls durch Fluoratome substituiert sein können. Beispielsweise seien genannt: Methylen, Fluormethylen, Difluormethylen, Äthylen, 1,2-Propylen, Äthyläthylen, Trimethylen, Tetramethylen, Pentamethylen, 1.1-Difluoräthylen, 1-Fluoräthylen, 1-Methyltetramethylen, 1-Methyl-tri-methylen, 1-Methylen-äthylen, 1-Methylen-tetra-methylen.

Zur Salzbildung sind anorganische und organische Basen geeignet, wie sie dem Fachmann zur Bildung physiologisch verträglicher Salze bekannt sind. Beispielsweise seien genannt Alkalihydroxide, wie Natrium- und Kaliumhydroxid, Erdalkalihydroxide, wie Calciumhydroxid, Ammoniak, Amine, wie Äthanolamin, Diäthanolamin, Triäthanolamin, N-Methylglucamin, Morpholin, Tris-(hydroxymethyl)-methylamin usw.

Die Erfindung betrifft ausserdem ein Verfahren zur Herstellung dsr erfindungsgemässen 9-Fluor-prostaglandinderivate der allgemeinen Formel I, dadurch gekennzeichnet, dass man in an sich bekannter Weise eine Verbindung der allgemeinen Formel II,

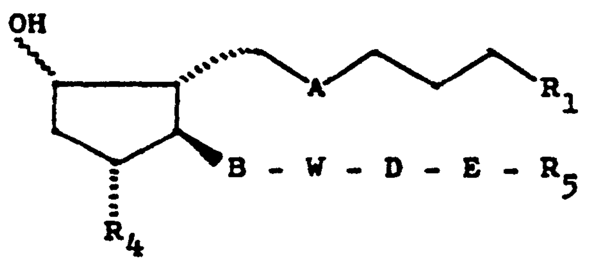

(II)

worin die OH-Gruppen α-oder β-ständig sein können, $R_1$ die Bedeutung der Reste

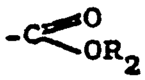

oder

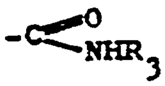

aufweist und A, B, D, E und $R_5$ die obenangegebenen Bedeutungen haben und freie OH-Gruppen in $R_4$ und W geschützt sind, über einen intermediären Sulfonsäureester mit einem Tetraalkylammoniumfluorid umsetzt und gegebenenfalls anschliessend in beliebiger Reihenfolge geschützte Hydroxygruppen freisetzt und/oder freie Hydroxygruppen verestert oder veräthert und/ oder Doppelbindungen hydriert, und/oder eine veresterte Carboxylgruppe ($R_1$ =

($R_1$ = 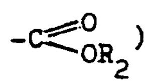 )

verseift und/oder eine Carboxylgruppe ($R_2$=H) verestert und/oder eine freie Carboxylgruppe ($R_2$=H) in ein Amid ($R_1$ =

($R_1$ = 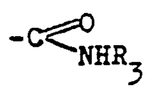 )

oder Salz überführt und/oder eine freie oder veresterte Carboxylgruppe ($R_1$ =

($R_1$ = 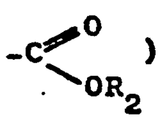 )

reduziert und/oder

falls $R_2$ die Bedeutung eines Wasserstoffatoms hat, die Überführung der Carboxylgruppe in Salze mit physiologisch verträglichen Basen.

Die Umsetzung der Verbindungen der allgemeinen Formel II zu einem Sulfonsäureester erfolgt in an sich bekannter Weise mit einem Alkyl- oder Arylsulfonylchlorid oder Alkyl- oder Arylsulfonsäureanhydrid in Gegenwart eines Amins wie beispielsweise Pyridin, 4-Dimethylaminopyridin oder Triäthylamin bei Temperaturen zwischen -60°C und + 100°C, vorzugsweise -20°C bis + 50°C. Die nucleophile Substition des 9-Sulfonats durch ein Flouratom erfolgt mit einem Tetraalkylammoniumfluorid, vorzugsweise Tetrabutylammoniumfluorid, in einem inerten Lösungsmittel wie beispielsweise Dimethylformamid, Dimethylsulfoxid, Dimethylacetamid, Dimethoxyäthan, Tetrahydrofuran Hexamethylphosphorsäuretriamid usw. bei Temperaturen zwischen 0°C und 80°C, vorzugsweise 20°C bis 45°C.

Setzt man bei der intermediären Sulfonesterbildung und nachfolgenden nucleophilen Substitution einen Alkohol der allgemeinen Formel II mit einer β-ständigen 9-Hy-droxygruppe ein, so erhält man Verbindungen der allgemeinen Formel I mit 9-α-ständigem Fluoratom, setzt man einen Alkohol mit einer α-ständigen Hydroxygruppe ein, so erhält man Verbindungen der allgemeinen Formel I mit 9-β-ständigem Fluoratom.

Die Reduktion zu den Verbindungen der allgemeinen Formel I mit $R_1$ in der Bedeutung einer $-CH_2OH$-Gruppe

wird mit einem für die Reduktion von Estern oder Carbonsäuren geeigneten Reduktionsmittel wie beispielsweise Lithiumaluminiumhydrid, Diisobutylaluminiumhydrid usw. durchgeführt. Als Lösungsmittel kommen Diäthyläther, Tetrahydroruran, Dimethoxyäthan, Toluol usw. in Frage. Die Reduktion wird bei Temperaturen von -30°C bis zur Siedetemperatur des verwendeten Lösungsmittels, vorzugsweise 0°C bis 30°C vorgenommen.

Die Freisetzung der funktionell abgewandelten Hydroxygruppen erfolgt nach bekannten Methoden. Beispielsweise wird die Abspaltung von Hydroxyschutzgruppen, wie beispielsweise des Tetrahydropyranylrestes, in einer wässrigen Lösung einer organischen Säure, wie z.B. Oxalsäure, Essigsäure, Propionsäure u.a., oder in einer wässrigen Lösung einer anorganischen Säure, wie z.B. Salzsäure, durchgeführt. zur Verbesserung der Löslichkeit wird zweckmässigerweise ein mit Wasser mischbares inertes organisches Lösungsmittel zugesetzt. Geeignete organische Lösungsmittel sind z.B. Alkohole, wie Methanol und Äthanol, und Äther, wie Dimethoxyäthan, Dioxan und Tetrahydrofuran. Tetrahydrofuran wird bevorzugt angewendet. Die Abspaltung wird vorzugsweise bei Temperaturen zwischen 20°C und 80°C durchgeführt.

Die Verseifung der Acylgruppen erfolgt beispielsweise mit Alkali- oder Erdalkali-carbonaten oder -hydroxyden in einem Alkohol oder in der wässrigen Lösung eines Alkohols. Als Alkohole kommen aliphatische Alkohole in Betracht, wie z.B. Methanol, Äthanol, Butanol usw., vorzugsweise Methanol. Als Alkalicarbonate und -hydroxyde seien Kalium- und Natriumsalze genannt. Bevorzugt sind die Kaliumsalze.

Als Erdalkalicarbonate und -hydroxyde sind beispielsweise geeignet Calciumcarbonat, Calciumhydroxyd und Bariumcarbonat. Die Umsetzung erfolgt bei -10°C bis + 70°C, vorzugsweise bei + 25°C.

Die Erfindung der Estergruppe

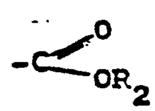

für $R_1$, bei welcher $R_2$ eine Alkylgruppe mit 1-4 C-Atomen darstellt, erfolgt nach den dem Fachmann bekannten Methoden. Die 1-Carboxy-verbindungen Herden beispielsweise mit Diazokohlenwasserstoffen in an sich bekannter Weise umgesetzt. Die Veresterung mit Diazokohlenwasserstoffen erfolgt z.B. dadurch, dass man eine Lösung des Diazokohlenwasserstoffes in einem inerten Lösungsmittel, vorzugsweise in Diäthyläther, mit der 1-Carboxyverbindung in dem gleichen oder in einem anderen inerten Lüsungsmittel, wie z.B. Methylenchlorid, vermischt. Nach beendeter Umsetzung in 1 bis 30 Minuten wird das Lösungsmittel entfernt und der Ester in üblicher Weise gereinigt. Diazoalkane sind, entweder bekannt oder können nach bekannten Methoden hergestellt werden [Og.Re,ctioas BC. 8, Seiten 389-394 (1954)].

Sollen im Primärprodukt enthaltene C=C-Doppelbindungen reduziert werden, erfolgt die Hydrierung nach an sich bekannten Methoden.

Die Hydrierung der 5,6-Doppelbindung wird in an sich bekannter Weise bei tiefen Temperaturen, vorzugsweise bei etwa -20°C, in einer Wasserstoffatmosphäre in Gegenwart eines Edelmetallkatalysators durchgeführt. Als Katalysator ist zum Beispiel 10% Palladium auf Kohle geeignet.

Werden sowohl die 5,6- als auch die 13,14-Doppelbindung hydriert, so arbeitet man bei höherer Temperatur vorzugsweise bei etwa 20°C.

Die Prostaglandinderivate der allgemeinen Formel I mit $R_2$ in der Bedeutung eines Wasserstoffatoms können mit geeigneten Mengen der entsprechenden anorganischen Basen unter Neutralisierung in ein Salz überführt werden. Beispielsweise erhält man beim Lösen der entsprechenden PG-Säurenin Wasser, das die stöchiometrische Menge der Base enthält, nach Abdampfen des Wassers oder nach Zugabe eines mit Wasser mischbaren Lösungsmittels, z.B. Alkohol oder Aceton, das feste anorganische Salz.

Zur Herstellung eines Aminsalzes, die in üblicher Weise erfolgt, wird die PG-Säure z.B. in einem geeigneten Lösungsmittel, beispielsweise Äthanol, Aceton, Diäthyläther, Acetonitril oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die Einführung der Amidgruppe

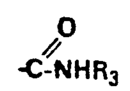

für $R_1$ erfolgt nach den dem Fachmann bekannten Methoden. Die Carbonsäuren der allgemeinen Formel 1 ($R_2$ = H), werden zunächst in Gegenwart eines tertiären Amins, wie beispielsweise Triäthylamin, mit Chlorameisensäureisobutylester in das gemischte Anhydrid überführt. Die Umsetzung des gemischten Anhydrids mit dem Alkalisalz des entsprechenden Amids oder mit Ammoniak ($R_3$ = H) erfolgt in einem inerten Lösungsmittel oder Lösungsmittelgemisch, wie beispielsweise Tetrahydrofuran, Dimethoxyäthan, Dimethylformamid, Hexamethylphosphorsäuretriamid, bei Temperaturen zwischen -30°C und + 60°C, vorzugsweise bei 0°C bis 30°C.

Eine weitere Möglichkeit für die Einführung der Amidgruppe

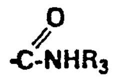

für $R_1$ besteht in der Umsetzung einer 1-Carbon-säure der allgemeinen Formel I ($R_2$=H), in der freie Hydroxygruppen gegebenenfalls intermediär geschützt sind, mit Verbindungen der allgemeinen Formel III,

$O = C = N - R_3$ (III),

worin $R_3$ die obenangegebene Bedeutung hat.

Die Umsetzung der Verbindung der allgemeinen Formel I ($R_2$=H) mit einem Isocyanat der allgemeinen Formel III erfolgt gegsbenenfalls unter Zusatz eines tertiären Amins, wie z.B. Triäthylamin oder Pyridin. Die Umsetzung kann ohne Lösungsmittel oder in einem inerten Lösungsmittel, vorzugsweise Acetonitril, Tetrahydrofuran, Aceton, Dimethylacetamid, Methylenchlorid, Diäthyläther, Toluol, bei Temperaturen zwischen -80°C bis 100°C, vorzugsweise bei 0 bis 30°C,vorgenommen werden.

Enthält das Ausgangsprodukt OH-Gruppen im Prostanrest, so werden diese OH-Gruppen auch zur Reaktion gebracht. Werden letztlich Endprodukte gewünscht, die freie Hydroxylgruppen im Prostanrest enthalten, geht man zweckmässigerweise von Ausgangsprodukten aus, in denen diese durch vorzugsweise leicht abspaltbare Äther- oder Acylreste intermediär geschützt sind.

Die als Ausgangsmaterial dienenden Verbindungen der allgemeinen Formel II können beispielsweise hergestellt werden, in dem man in an sich bekannter Weise ein Keton der allgemeinen Formel IV

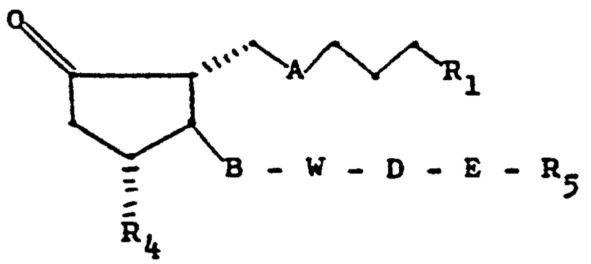

(IV)

worin $R_1$ die Reste

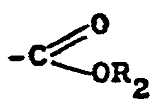

oder

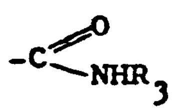

bedeutet und

A, B, D, E und $R_5$ die obenangegebenen Bedeutungen haben und freie OH-Gruppen in $R_4$ und W geschützt sind, mit Natriumborhydrid, Lithium-tris(tert.-butoxy)aluminiumhydrid usw. reduziert und gebenenfalls anschliessend die epimeren 9α- und 9β-ständigen Hydroxyverbindungen der allgemeinen Formel II trennt.

Im Vergleich zu PGE-Derivaten zeichnen sich die neuen 9-Fluor-prostaglandine durch grössere Stabilität aus.

Die neuen 9-Fluor-prostanderivate der allgemeinen Formel I sind wertvolle Pharmaka, da sie bei ähnlichem Wirkungsspektrum eine wesentlich verbesserte (höhere Spezifität) und vor allem wesentlich längere Wirkung aufweisen als die entsprechenden natürlichen Prostaglandine.

Die neuen Prostaglandin-Analoga wirken stark luteolytisch, d.h. zur Auslösung einer Luteolyse benötigt man

wesentlich geringere Dosierungen als bei den entsprechenden natürlichen Prostaglandinen.

Auch zur Auslösung von Aborten, insbesondere nach oraler oder intravaginaler Applikation, sind wesentlich geringere Mengen der neuen Prostaglandinanaloga im Vergleich zu den natürlichen Prostaglandinen erforderlich.

Bei der Registrierung der isotonischen Uteruskontraktion an der narkotisierten Ratte und am isolierten Rattenuterus zeigt sich, dass die erfindungsgemässen Substanzen wesentlich wirksamer sind und ihre Wirkungen länger anhalten als bei den natürlichen Prostaglandinen.

Die neuen Prostaglandinderivate sind geeignet, nach einmaliger enteraler oder parenteraler Applikation eine Menstruation zu induzieren oder eine Schwangerschaft zu unterbrechen. Sie eignen sich ferner zur Synchronisation des Sexualzyklus bei weiblichen Säugetieren wie Kaninchen, Rindern, Pferden, Schweinen usw. Ferner eignen sich die erfindungsgemässen Prostaglandin-Derivate zur Cervixdilatation als Vorbereitung für diagnostische oder therapeutische Eingriffe.

Die gute Gewebsspezifität der erfindungsgemässen antifertil wirksamen Substanzen zeigt sich bei der Untersuchung an anderen glattmuskulären Organen, wie beispielsweise am Meerschweinchen-Ileum oder an der isolierten Kaninchen-Trachea, wo eine wesentlich geringere Stimulierung zu beobachten ist als durch die natürlichen Prostaglandine. Die erfindungsgemässen Substanzen wirken auch bronchospasmolytisch. Ausserdem bewirken sie eine Abschwellung der Nasenschleimhaut.

Die erfindungsgemässen Wirkstoffe hemmen die Magensäuresekretion, zeigen einen zytoprotektiven und ulcusheilenden Effekt und wirken damit den unerwünschten Folgen nichtsteroidaler Entzündungshemmstoffe (Prostaglandinsynthese - Inhibitoren) entgegen. Sie wirken ausserdem an der Leber und auch an der Bauchspeicheldrüse zytoprotektiv.

Einige der Verbindungen wirken blutdrucksenkend, regulierend by Herzrhythmusstörungen und hemmend auf die Plättchenaggregation mit den sich daraus ergebenden Einsatzmöglichkeiten. Die neuen Prostaglandine können auch in Kombination z.B. mit β-Blockern und Diuretika verwendet werden.

Für die medizinische Anwendung können die Wirkstoffe in eine für die Inhalation, für orale, parenterale oder lokale (z.B. vaginale) Applikation geeignets form überführt werden.

Zur Inhalation werden zweckmässigerweise Arosollösungen hergestellt.

Für die orale Applikation sind beispielsweise Tabletten, Dragees oder Kapseln geeignet.

Für die parenterale Verabreichung werden sterile injizierbare, wässrige oder ölige Lösungen benutzt.

Für die vaginale Applikation sind z.B. Zäpfchen geeignet und üblich.

Die Erfindung betrifft damit auch Arzneimittel auf Basis der Verbindungen der allgemeinen Formel I und der üblichen Hilfs- und Trägerstoffe.

Die erfindungsgemässen Wirkstoffe sollen in Verbindung mit den in der Galenik bekannten und üblichen Hilfsstoffen, z.B. zur Herstellung von Präparaten zur Auslösung eines Abortes, zur Zyklussteuerung, zur Einleitung einer Geburt oder zur Behandlung der Hypertonie dienen. Für diesen Zweck aber auch für die übrigen Anwsndungen können die Präparate 0,01 - 50 mg der aktiven Verbindung enthalten.

Die folgenden Beispiele sollen die Erfindung näher erläutern, ohne dass damit eine Begrenzung vorgenommen wird.

**Beispiel 1**

(5Z, 13E)-(9R, 11R, 15R)-11, 15-Dihydroxy-9-fluor-16-phenoxy-17, 18, 19, 20-tetranor-5, 13-prostadiensäuremethylester

Zu einer Lösung von 5,7 g (5Z, 13E)-(9S, 11R, 15R)-9-Hydroxy-11,15-bis-(tetrahydropyran-2-yloxy)-16-phenoxy-17, 18, 19, 20-tetranor-5, 13-prostadiensäuremethylester (hergestellt aus der entsprechenden Säure in Methylenchlorid mit 0,5 molarer Diazomethanlösung bei 0°C) in 18 ml Pyridin fügt man bei 0°C 3,8 g p-Toluolsulfonsäurechlorid, rührt 16 Stunden bei Eisbadtemperatur und 48 Stunden bei Raumtemperatur. Anschließend versetzt man mit 15 ml Wasser, rührt 3 Stunden bei 20°C, verdünnt mit Äther, schüttelt nacheinander mit Wasser, 5%iger Schwefelsäure, 5%iger Natriumbicarbonatlösung und Wasser. Man trocknet über Magnesiumsulfat und dampft im Vakuum ein. Nach Chromatographie des Rückstandes an Kieselgel erhält man mit Hexan/Essigester (3+2) 5,2 g des 9-Tosylats als farbloses Öl. IR: 2950, 2875, 1735, 1600, 1590, 1496, 1365, 1240, 974/cm.

Zu einer Lösung von 5,2 g des vorstehend erhaltenen 9-Tosylats in 75 ml abs. Dimethylsulfoxid fügt man 13 g Tetrabutylammoniumfluorid (getrocknet durch mehrmaliges Einengen mit Toluol) und rührt die Lösung 1,5 Stunden bei 22°C unter Argon. Anschließend verdünnt man mit Äther, schüttelt viermal mit Wasser, trocknet über Magnesiumsulfat und dampft im Vakuum ein. Den Rückstand reinigt man durch Chromatographie an Kieselgel. Mit Hexan/Essigester (9+1) als Elutionsmittel erhält man zunächst die entsprechende $\Delta^{8,9}$-Verbindung sowie als polarere Komponente 0,72 g (5Z, 13Z)-(9R, 11R, 15R)-11, 15-Bis-(tetrahydropyran-2-yloxy)-9-fluor-16-phenoxy-17, 18, 19, 20-tetranor-5, 13-prostadiensäuremethylester als farbloses Öl.

IR: 2955, 1733, 1600, 1588, 1495, 970/cm.

Zur Schutzgruppenabspaltung rührt man 0,72 g der vorstehend erhaltenen 9β-Fluorverbindung 16 Stunden bei 22°C mit 20 ml einer Mischung aus Essigsäure, Wasser, Tetrahydrofuran (65+35+10) und dampft anschließend im Vakuum ein. Den Rückstand reinigt man durch Chromatographie an Kieselgel. Mit Äther als Elutionsmittel erhält man 370 mg der Titelverbindung als farbloses Öl.

IR: 3600, 3430 (breit), 2952, 2950, 2870, 1750, 1600, 1588, 1495, 970/cm.

**Beispiel 2**

(5Z, 13E)-(9S, 11R, 15R)-11, 15-Dihydroxy-9-fluor-16-Phenoxy-17, 18, 19, 20-tetranor-5,13-prostadiensäuremethylester

Zu einer Lösung von 4,8 g (5Z, 15E)-(9R, 11R, 15R)-9-Hydroxy-11, 15-bis-(tetrahydropyran-2-yloxy)-16-Phenoxy-17, 18, 19, 20-tetranor-5, 13-prostadiensäuremethylester (hergestellt aus der entsprechenden Säure in Methylenchlorid mit 0,5 molarer ätherischer Diazomethanlösung bei 0°C) in 27 ml Pyridin fügt man bei 20°0 3,4 g P-Toluolsulfonsäurechlorid und rührt 20 Stunden bei 20°C. Anschließend versetzt man mit 12 ml Wasser, rührt 5 Stunden bei 20°C, verdünnt mit Äther, schüttelt nacheinander mit Wasser, 5%iger Schwefelsäure, 5%iger Natriumbicarbonatlösung und Wasser. Man trocknet über Magnesiumsulfat und dampft im Vakuum ein. Dabei erhält man 6,05 g des 9-Tosylats als farbloses Öl.

IR: 2950, 2875, 1730, 1599, 1588, 1495, 1360, 972/cm.

Zu einer Lösung von 6,05 g des vorstehend erhaltenen 9-Tosylats in 180 ml abs. Tetrahydroruran fügt man 13,3 g Tetrabutylammoniumfluorid (getrocknet durch mehrmaliges Einengen mit Toluol) und kocht die Lösung 1 Stunde am Rückfluß unter Argon. Anschließend verdünnt man mit Äther, schüttelt viermal mit Wasser, trocknet über Magnesiumsulfat und dampft im Vakuum ein. Den Rückstand reinigt man durch Chromatographie an Kieselgel. Mit Hexan/Äther (7+5) erhält man 2,2 g (5Z, 15E)-(9S, 11R, 15R)-11, 15-Bis-(tetrahydropyran-2-yloxy)-9-fluor-16-phenoxy-17, 18, 19, 20-tetranor-5, 13-Prostadien-säuremethylester als farbloses Öl.

IR: 2956, 1731, 1599, 1588, 1495, 972/cm.

Zur Schutzgruppenspaltung rührt man 2,2 g der vorstehend erhaltenen 9α-Fluorverbindung 16 Stunden mit 90 ml einer Mischung aus Essigsäure/Wasser/Tetrahydrofuran (65+35+10) bei 20°C und dampft anschließend im Vakuum ein. Den Rückstand reinigt man durch Chromatographie an Kieselgel. Mit Äther als Elutionsmittel erhält man 1,45 g der Titelverbindung als farbloses Öl.

IR: 3600, 3420 (breit), 2930, 2860, 1730, 1600, 1589, 1434, 970/cm.

**Beispiel 3**

(13E)-(9R, 11R, 15R)-11, 15-Dihydroxy-9-fluor-16-phenoxy-17, 18, 19, 20-tetranor-13-Prostensäuremethylester

In Analogie zu Beispiel 1 erhält man aus 2,5 g (13E)-(9S, 11R, 15R)-9-Hydroxy-11, 15-bis-(tetrahydropyran-2-yloxy)-16-Phenoxy-17, 18, 19, 20-tetranor-13-prostensäure-methylester 0,39 g (13E)-(9R, 11R, 15R)-11, 15-Bis-(tetrahydropyran-2-yloxy)-9-fluor-16-Phenoxy-17, 18, 19, 20-tetranor-13-prostensäuremethylester als farbloses Öl.

IR: 2956, 1732, 1600, 1588, 1495, 970/cm.

Nach Abspaltung der Schutzgruppen gemäß Beispiel 1 erhält man 0,24 g der Titelverbindung als farbloses Öl.

IR: 3600, 3440 (breit), 2953, 2950, 2870, 1730, 1600, 1588, 1495, 970/cm.

**Beispiel 4**

(15E)-(9S, 11R, 15R)-11, 15-Dihydroxy-9-fluor-16-phenoxy-17, 18, 19, 20-tetranor-13-prostensäuremethylester

In Analogie zu Beispiel 2 erhält man aus 2,1 g (13E)-(9R, 11R, 15R)-9-Hydroxy-11, 15-bis-(tetrahydropyran-2-yloxy)-16-phenoxy-17, 18, 19, 20-tetranor-13-prostensäuremethylester 0,9 g (13E)-(9S, 11R, 15R)-11, 15-Bis-(tetrahydropyran-2-yloxy)-9-fluor-16-phenoxy-17, 18, 19, 20-tetranor-13-prostensäuremethylester als farbloses Öl. IR: 2955, 1732, 1599, 1588, 1495, 971/cm.

Nach Abspaltung der Schutzgruppen gemäß Beispiel 1 erhält man 0,52 g der Titelverbindung als farbloses Öl.

IR: 3600, 3450 (breit), 2930, 2860, 1751, 1600, 1589, 1494, 970/cm.

**Beispiel 5**

(5Z, 13E)-(9R, 11R, 15R)-11,15-Dihydroxy-16,16-dimethyl-9-fluor-5, 13-prostadiensäuremethylester

Im Analogie zu Beispiel 1 erhält man aus 2,9 g (5Z, 13E)-(9S, 11R, 15R)-11, 15-Eis-(tetrahydropyran-2-yloxy)-16, 16-dimethyl-9-hydroxy-5, 13-prostadiensäuremethylester 0,35 g (5Z, 15E)-(9R, 11R, 15R)-11, 15-Eis-(tetrahydropyran-2-yloxy-16, 16-dimethyl-9-fluor-5, 13-prosta-diensäuremethylester als farbloses Öl.

IR: 2960, 2855, 1732, 974/cm.

Nach Abspaltung der Schutzgruppen gemäß Beispiel 1 erhält man 0,23 g der Titelverbindung als farbloses Öl.

IR: 3610, 3400 (breit), 2940, 2860, 1732, 974/cm.

**Beispiel 6**

(5Z, 15E)-(9S, 11R, 15R)-11, 15-Dihydroxy-16, 16-dimethyl-9-fluor-5, 13-prostadiensäuremethylester

In Analogie zu Beispiel 2 erhält man aus 1 g (5Z, 13E)-(9R, 11R, 15R)-9-Hydroxy-11, 15-Bis-(tetrahydropyran-2-yloxy)16, 16-dimethyl-5, 13-prostadiensäuremethylester 0,44 g (5Z, 13E)-(9S, 11R, 15R)-11, 15-Bis-(tetrahydropyran-2-yloxy)-16, 16-dimethyl-9-fluor-5, 13-prosta-diensäuremethylester als Öl.

IR: 2960, 2856, 1731, 974/cm.

Nach Abspaltung der Schutzgruppen gemäß Beispiel 2 erhält man 0,28 g der Titelverbindung als Öl.

IR: 3600, 3440 (breit), 2935, 2860, 1732, 974/cm.

Das Ausgangsmaterial für die obige Titelverbindung wird wie folgt hergestellt:

6a) (5Z, 13E)-(9R, 11R, 15R)-9-Hydroxy-11, 15-bis-(tetrahydropyran-2-yloxy)-16, 16-dimethyl-5, 13-prostadiensäuremethylester

Zu einer Lösung von 18g (5Z, 15E)-(11R, 15R)-11, 15-bis-(tetra-hydropyran-2-yloxy)-16, 16-dimethyl-9-oxo-5, 13-Prostadien-säuremethylester in 400 ml Methanol fügt man bei 0°C 6,5 g Natriumborhydrid und rührt 30 Minuten bei 0°C. Anschließend verdünnt man mit Äther, schüttelt viermal mit Wasser, trocknet über Magnesiumsulfat und dampft im Vakuum ein. Den Rückstand reinigt man durch Säulenchromatographie an Kieselgel. Hit Äther/Hexan (4+1) als Elutionsmittel erhält man zunächst 6,8 g der entsprechenden 9α-Hydroxyverbindung sowie als polarere Komponente 6,05 g der Titelverbindung als farbloses Öl.

IR: 5600, 3420 (breit), 2960, 2855, 1731, 972/cm.

**Beispiel 7**

(13E)-(9R, 11R, 15R)-11, 15-Dihydroxy-16, 16-dimethyl-9-fluor-13-prostensäuremethylester

In Analogie zu Beispiel 1 erhält man aus 1,5 g (13E)-(9S, 11R, 15R)-11, 15-Bis-(tetrahydropyran-2-yloxy)-16, 16-dimethyl-9-hydroxy-13-prostensäuremethylester 180 mg (13E)-(9R, 11R, 15R)-11, 15-Bis-(tetrahydropyran-2-yloxy)-16, 16-dimethyl-9-fluor-13-prostensäure-methylester als Öl.

IR: 2962, 2855, 1731, 972/cm.

Nach Abspaltung der Schutzgruppen gemäß Beispiel 1 erhält man 105 mg der Titelverbindung als farbloses Öl.

IR: 3600, 5440 (breit), 2955, 2860, 1731, 972/cm.

**Beispiel 8**

(5Z, 13E)-(9R, 11R, 16RS)-11, 15α-Dihydroxy-9-fluor-16-methyl-5, 15-prostadiensäuremethylester

In Analogie zu Beispiel 1 erhält man aus 3 g (5Z, 13E)-(9S, 11R, 16RS)-11, 15α-Bis-(tetrahydropyran-2-yloxy)-9-hydroxy-16-methyl-5, 13-Prostadiensäuremethylester 0,53 g (5Z, 15E)-(9R, 11R, 16RS)-11,15α-Bis-(tetrahydropyram-2-yloxy)-9-fluor-16-methyl-5, 13-prostadiensäuremethylester als farbloses Öl.

IR: 2965, 2855, 1732, 970/cm.

Nach Abspaltung der Schutzgruppen gemäß Beispiel 1 erhält man 0,2 g der Titelverbindung als Öl.

IR: 3600, 3430 (breit), 2935, 2860, 1752, 970/cm.

**Beispiel 9**

(5Z, 13E)-(9S, 11R, 16RS)-11, 15α-Dihydroxy-9-fluor-16-methyl-5, 13-prostadiensäuremethylester

In Analogie zu Beispiel 2 erhält man aus 1,5 g (5Z, 13E)-(9R, 11R, l6RS)-11, 15α-Bis-(tetrahydropyran-2-yloxy)-9-hydroxy-16-methyl-5, 13-prostadiensäuremethylester 0,58 g (5Z, 13E)-(9S, 11R, 16RS)-11,15α-Bis-(tetrahydropyran-2-yloxy)-9-fluor-16-methyl-5, 13-prostadiensäuremethylester als farbloses Öl.

IR: 2966, 2858, 1732, 971/cm.

Nach Abspaltung der Schutzgruppen gemäß Beispiel 2 erhält man 0,45 g der Titelverbindung als Öl.

IR: 3600, 5440 (breit), 2955, 2860, 1732, 971/cm.

Das Ausgangsmaterial für die obige Titelverbindung wird wie folgt hergestellt:

9a) (5Z, 15E)-(9R, 11R, 16RS)-11, 15α-Bis-(tetrahydropyran-2-yloxy)-9-hydroxy-16-methyl-5,13-prostadiensäuremethylester

In Analogie zu Beispiel 6a erhält man aus 7g (5Z, 13E)-(IIR, 16RS)-11, 15α-bis-(tetrahydropyran-2-yloxy)-16-methyl-9-oxo-5, 13-prostadiensäuremethylester 2,4 g der Titelverbindung als Öl.

IR: 3610, 3440 (breit), 2960, 2856, 1732, 971/cm.

**Beispiel 10**

(5Z, 13E)-(9R, 11R, 15R, 16RS)-11, 15-Dihydroxy-9, 16-difluor-5, 13-prostadiensäuremethylester

In Analogie zu Beispiel 1 erhält man aus 4,2 g (5Z,13E)-(9S, 11R, 15R, 16RS)-11, 15-Bis-(tetrahydropyran-2-yloxy)-9-hydroxy-16-fluor-5, 15-prostadiensäuremethylester 490 mg (5Z, 13E)-(9S, 11R, 15R, 16RS)-11, 15-Bis-(tetrahydropyran-2-yloxy)-9,16-difluor-5, 13-prostadien-säuremethylester als Öl.

IR: 2960, 1755, 976/cm.

Nach Abspaltung der Schutzgruppen gemäß BeisPiel 1 erhält man 285 mg der Titelverbindung als Öl.

IR: 3600, 3430 (breit), 2930, 2857, 1734, 976/cm.

**Beispiel 11**

(5Z, 13E)-(9S, 11R, 15R, 16RS)-11, 15-Dihydroxy-9, 16-difluor-5, 13-prostadiensäuremethylester

In Analogie zu Beispiel 2 erhält man aus 0,8 g (5Z, 13E)-(9R, 11R, 15R, 16RS)-11, 15-Bis-(tetrahydropyran-2-yloxy)-9-hydroxy-16-fluor-5, 13-prostadiensäuremethylester (hergestellt aus dem entsprechenden 9-Keton gemäß Beispiel 6a) 0,37 g (5Z, 13E)-(9S, 11R, 15R, 16RS)-11, 15-Bis-(tetrahydropyran-2-yloxy)-9, 16-difluor-5, 13-prostadiensäuremethylester als farbloses Öl.

IR: 2958, 1754, 974/cm.

Nach Abspaltumg der Schutzgruppen gemäß Beispiel 2 erhält man 240 mg der Titelverbindung als Öl.

IR: 3600, 3440 (breit), 2932, 2856, 1732, 974/cm.

**Beispiel 12**

(5Z, 13E)-(9R, 11R, 16RS)-11, 15α-Dihydroxy-16, 19-dimethyl-9-fluor-5, 13, 18-prostatriensäuremethylester

In Analogie zu Beispiel 1 erhält man 2,8 g (5Z, 13E)-(9S, 11R, 16RS)-11, 15α-Bis-(tetrahydropyran-2-yloxy)-16, 19-dimethyl-9-hydroxy-5, 13, 18-prostatriensäuremethylester 0,38 g (5Z, 13E)-(9R, 11R, 16RS)-11, 15α-Bis-(tetrahydropyran-2-yloxy)-16, 19-dimethyl-9-fluor-5, 15, 18-prostatriensäuremethylester als farbloses Öl.

IR: 2960, 1731, 972/cm.

Nach Abspaltung der Schutzgruppen gemäß Beispiel 1 erhält man 0,29 g der Titelverbindung als Öl.

IR: 5600, 3440 (breit), 2935, 2855, 1731, 972/cm.

**Beispiel 13**

(5Z, 13E)-(9S, 11R, 16RS)-11, 15 α-Dihydroxy-16, 19-dimethyl-9-fluor-5, 13, 18-prostatriensäuremethylester

In Analogie zu Beispiel 2 erhält man aus 0,6 g (5Z, 13E)-(9R, 11R, l6RS)-11, 15 α-Bis-(tetrahydropyran-2-yloxy)-16, 19-dimethyl-9-hydroxy-5, 13, 18-prostatriensäuremethylester 0,24 g (5Z, 13E)-(9S, 11R 16RS)-11,15 α-Bis-(tetrahydropyran-2-yloxy)-16,19-dimethyl-9-fluor-5, 13, 18-prostatriensäuremethylester als farbloses Öl.

IR: 2962, 1732, 970/cm.

Nach Abspaltung der Schutzgruppen gemäß Beispiel 1 erhält man 140 mg der Titelverbindung als Öl.

IR: 3600, 3420 (breit), 2935, 2856, 1731, 970/cm.

Das Ausgangsmaterial für die obige Titelverbindung wird wie folgt hergestellt:

13a) (5Z, 13E)-(9R, 11R, 16RS)-11, 15α-Bis-(tetrahydropyran-2-yloxy)-16, 19-dimethyl-9-hydroxy-5, 13, 18-prostatriensäuremethylester

In Amalogie zu Beispiel 6a erhält man aus 4,2 g (5Z, 13E)-(11R, 16RS)-11, 15α-Bis-(tetrahydropyran-2-yloxy)-16, 19-dimethyl-9-oxo-5, 15, 18-prostatriensäuremethylester (hergestellt aus der entsprechenden Säure mit ätherischer Diazomethanlösung bei 0°C) 1,7 g der Titelverbindung als farbloses Öl.

IR: 3600, 5420 (breit), 2965, 2855, 1733, 970/cm.

**Beispiel 14**

(15E)-(9R, 11R, 16RS)-11,15α-Dihydroxy-16, 19-dimethyl-9-fluor-15, 18-prostadiensäuremethylester

In Analogie zu Beispiel 1 erhält man aus 1,3 g (13E)-(9S, 11R, 16RS)-11, 15α-Bis-(tetrahydropyran-2-yloxy)-16, 19-dimethyl-9-hydroxy-13, 18-prostadiensäuremethylester 170 mg (13E)-(9R, 11R, 16RS)-11, 15α-Bis-(tetrahydropyran-2-yloxy)-16, 19-dimethyl-9-fluor-13, 18-prosta-diensäuremethylester als farbloses Öl.

IR: 2962, 1730, 971/cm.

Nach Abspaltung der Schutzgruppen gemäß Beispiel 1 erhält man 95 mg der Titelverbindung als farbloses Öl.

IR: 3610, 5450 (breit), 2957, 2855, 1750, 971/cm.

**Beispiel 15**

(15E)-(9S, 11R, 16RS)-11, 15α-Dihydroxy-16, 19-dimethyl-9-fluor-13, 18-prostadiensäuremethylester

In Analogie zu Beispiel 2 erhält man aus 0,9 g (13E)-(9R, 11R, 16RS)-11, 15α-Bis-(tetrahydropyran-2-yloxy)-16, 19-dimethyl-9-hydroxy-15, 18-prostadiensäuremethylester 0,58 g (15E)-(9S, 11R, 16RS)-11, 5 α-Bis-(tetrahydropyran-2-yloxy)-16, 19-dimethyl-9-fluor-13, 18-prosta-diensäuremethylester als farbloses Öl.

IR: 2960, 1731, 970/cm.

Nach Abspaltung der Schutzgruppen gemäß Beispiel 2 erhält man 220 mg der Titelverbindung als farbloses Öl.

IR: 3600, 3440 (breit), 2936, 2855, 1731, 970/cm.

Das Ausgangsmaterial für die obige Titelverbindung wird wie folgt hergestellt:

15a) (15E)-(9R, 11R, 16RS)-11, 15α-Bis-(tetrahydropyran-2-yloxy)-16, 19-dimethyl-9-hydroxy-13, 18-prostadiensäuremethylester

In Analogie zu Beispiel 6a erhält man aus 5,1 g (13E)-(11R, 16RS)-11, 15α-Bis-(tetrahydropyran-2-yloxy)-16, 19-dimethyl-9-oxo-13, 18-prostadiennäuremethylester (hergestellt aus der entsprechenden Säure mit ätherischer Diazomethanlösung,bei 0°C) 2,2 g der Titelverbindung als farbloses Öl.

IR: 3600, 3440 (breit), 2963, 2855, 1732, 970/cm.

**Beispiel 16**

(5Z, 13E)-(9R, 11R, 15R)-11, 15-Dihydroxy-9-fluor-16, 16, 19-trimethyl-5, 13, 18-prostatriensäuremethylester

In Analogie zu Beispiel 1 erhält man aus 2,9 g (5Z, 13E)-(9S, 11R, 15R)-11, 15-Bis-(tetrahydropyran-2-yloxy)-9-hydroxy-16, 16, 19-tri-methyl-5, 15, 18-prostatriensäuremethylester 350 mg (5Z, 13E)-(9R, 11R, 15R)-11, 15-Bis-(tetrahydropyran-2-yloxy)-9-fluor-16, 16, 19-trimetyl-5, 13, 18-prostatriensäuremetylester als farbloses Öl.

IR: 2960, 2855, 1732, 974/cm

Nach abspaltung der Schutzgruppe gemäß Beispiel 1 erhält man 190 mg der Titelverbindung als Öl.

IR: 3600, 3440 (breit), 2942, 2850, 1732, 973/cm

**Beispiel 17**

(5Z, 13E)-(9R, 11R, 15R)-11, 15-Dihydroxy-9-fluor-16-phenoxy-17, 18, 19, 20-tetranor-5, 13-prostadiensäure

350 mg des nach Beispiel 1 hergestellten Methylesters rührt man 5 Stunden mit 10 ml einer Lösung aus Kaliumhydroxid in Äthanol und Wasser (Herstellung: Man löst 2g Kaliumhydroxid in 75 ml Äthanol und 25 ml Wasser). Anschließend säuert man mit 10%iger Zitronensäurelösung auf pH4 an, extrahiert dreimal mit Methylenchlorid, wäscht den organischen Extrakt einmal mit Sole, trocknet über Magnesiumsulfat und dampft im Vakuum ein. Nach Chromatographie des Rückstandes an Kieselgel mit Methylenchlorid/Isopropanol (9+1) als Elutionsmittel erhält man 300 mg ier Titelverbindung als farbloses Öl.

IR: 3580, 3400 (breit), 2925, 2865, 1710, 1598, 1588, 1493, 970/cm.

**Beispiel 18**

(5Z, 13E)-(9S, 11R, 15R)-11, 15-Dihydroxy-9-fluor-16-phenoxy-17, 18, 19, 20-tetranor-5, 13-prostadiensäure

In Analogie zu Beispiel 17 erhält man aus 0,42 g des nach Beispiel 2 hergestellten Methylesters 0,54 g der Titelverbindung als Öl.

IR: 5590, 5410 (breit), 2925, 2865, 1708, 1598, 1588, 1493, 970/cm.

**Beispiel 19**

(13E)-(9R, 11R, 15R)-11, 15-Dihydroxy-9-fluor-16-phenoxy-17, 18, 19, 20-tetranor-13-prostensäure

In Analogie zu Beispiel 17 erhält man aus 0,26 g des nach Beispiel 3 hergestellten Methylesters 0,20 g der Titelverbindung als farblose Kristalle (F.98-99°C).

IR: 3600, 3400 (breit), 2924, 2864, 1709, 1599, 1588, 1493, 971/cm.

**Beispiel 20**

(13E)-(9S, 11R, 15R)-11, 15-Dihydroxy-9-fluor-16-phenoxy-17, 18, 19, 20-tetranor-13-prostensaure

In Analogie zu Beispiel 17 erhält man aus 0,32 g des nach Beispiel 4 hergestellten Methylesters 0,26 g der Titelverbindung als farbloses Kristalle (F.86-87°C).

IR: 3590, 3400 (breit), 2924, 2865, 1708, 1598, 1588, 1493, 970/cm.

**Beispiel 21**

(5Z, 13E)-(9R, 11R, 15R)-11, 15-Dihydroxy-16, 16-dimethyl-9-fluor-5, 13-prostadiensäure

In Analogie zu Beispiel 17 erhält man aus 0,15 g des nach Beispiel 5 hergestellten Methylesters 0,11g der Titelverbindung als Öl.

IR: 3600, 3405 (breit), 2952, 2860, 1710, 974/cm.

**Beispiel 22**

(5Z, 13E)-(9S, 11R, 15R)-11, 15-Dihydroxy-16, 16-dimethyl-9-fluor-5,13-prostadiensäure

In Analogie zu Beispiel 17 erhält man aus 0,15 g des nach Beispiel 6 hergestellten Methylesters 0,10 g der Titelverbindung als Öl.

IR: 3600, 3405 (breit), 2950, 2858, 1710, 975/cm.

**Beispiel 23**

(13E)-(9R, 11R, 15R)-11, 15-Dihydroxy-16, 16-dimethyl-9-fluor-13-prostensäure

In Analogie zu Beispiel 17 erhält man aus 0,2 g des nach Beispiel 7 hergestellten Methylesters 0,16g der Titelverbindung als Öl.

IR: 3600, 3410 (breit), 2952, 2862, 1708, 974/cm.

**Beispiel 24**

(5Z, 13E)-(9R, 11R, 16RS)-11, 15α-Dihydroxy-9-fluor-16-methyl-5, 13-prostadiensäure

In Analogie zu Beispiel 17 erhält man aus 0,2 g des nach Beispiel 8 hergestellten Methylesters 0,16 g der Titelverbindung als Öl.

IR: 3600, 3405 (breit), 2945, 2848, 1710, 976/cm.

**Beispiel 25**

(5Z, 13E)-(9S, 11R, 16RS)-11, 15 α-Dihydroxy-9-fluor-16-methyl-5, 13-prostadiensäure

In Analogie zu Beispiel 17 erhält man aus 0,2 g des nach Beispiel 9 hergestellten Methylesters 0,17 g der Titelverbindung als Öl.

IR: 3600, 3405 (breit), 2950, 2852, 1710, 976/cm.

**Beispiel 26**

(5Z, 13E)-(9R, 11R, 15R, 16RS)-11, 15-Dihydroxy-9, 16-difluor-5, 13-prostadiensäure

In Analogie zu Beispiel 17 erhält man aus 0,15 g des nach Beispiel 10 hergestellten Methylesters 0,12 g der Titelverbindung als Öl.

IR: 3600, 3410 (breit), 2955, 2860, 1708, 976/cm.

**Beispiel 27**

(5Z, 13E)-(9S, 11R, 15R, 16RS)-11, 15-Dihydroxy-9, 16-difluor-5, 13-prostadiensäure

In Analogie zu Beispiel 17 erhält man aus 0,15 g des nach Beispiel 11 hergestellten Methylesters 0,11 g der Titelverbindung als Öl.

IR: 3600, 3405 (breit), 2954, 2858, 1708, 974/cm.

**Beispiel 28**

(5Z, 13E)-(9R, 11R, 16RS)-11, 15 α-Dihydroxy-16, 19-dimethyl-9-fluor 5, 13, 18-prostatriensäure

In Analogie zu Beispiel 17 erhält man aus 0,30 g des nach Beispiel 12 hergestellten Methylesters 0,26 g der Titelverbindung als Öl.

IR: 3600, 3410 (breit), 2938, 2856, 1710, 976/cm.

**Beispiel 29**

(5Z, 13E)-(9S, 11R, 16RS)-11, 15α-Dihydroxy-16, 19,-dimethyl-9-fluor-5, 13, 18-prostatriensäure

In Analogie zu Beispiel 17 erhält man aus 90 mg des nach Beispiel 13 hergestellten Methylesters 75 mg der Titelverbindung als Öl.

IR: 3600, 3410 (breit), 2940, 2856, 1710, 974/cm.

**Beispiel 30**

(13E)-(9R, 11R, 16RS)-11, 15 α-Dihydroxy-16, 19-dimethyl-9-fluor-13, 18-prostadiensäure

In Analogie zu Beispiel 17 erhält man aus 80 mg des nach Beispiel 14 hergestellten Methylesters 50 mg der Titelverbindung als Öl.

IR: 3600, 3405 (breit), 2952, 2840, 1710, 974/cm.

**Beispiel 31**

(13E)-(9S, 11R, 16RS)-11, 15α-Dihydroxy-16, 19-dimethyl-9-fluor-13, 18-prostadiensaure

In Analogie zu Beispiel 17 erhält man aus 150 mg des nach Beispiel 15 hergestellten Methylesters 110 mg der Titelverbindung als Öl.

IR: 3600, 3405 (breit), 2940, 2862, 1710, 974/cm.

**Beispiel 32**

(5Z, 13E)-(9R, 11R, 15R)-11, 15-Dihydroxy-9-fluor-16, 16, 19-trimethyl-5, 13, 18-prostatriensäure

In Analogie zu Beispiel 17 erhält man aus 100 mg des nach Beispiel 16 hergestellten Methylesters 70 mg der Titelverbindung als Öl.

IR: 3600, 3400 (breit), 2958, 2850, 1710, 976/cm.

**Beispiel 33**

(13E)-(9R, 11R, 15R)-9-Fluor-1, 11, 15-trihydroxy-16-phenoxy-17, 18, 19, 20-tetranor-13-prosten

Zu einer lösung von 200 mg (13E)-(9R, 11R, 15R)-11, 15-Dihydroxy-9-fluor-16-phenoxy-17, 18, 19, 20-tetranor-13-prostensäuremethyl-ester (hergestellt nach Beispiel 3) in 10 ml Tetrahydrofuran fügt man bei 0°C unter Rühren portionsweise 300 mg Lithiumaluminiunhydrid und rührt anschließend 30 Minuten bei 25°C. Man zerstört den Reagenzüberschuß bei 0°C durch tropfenweise Zugabe von Essigester, fügt 2 ml Wasser und 60 ml Äther zu und rührt intensiv 3 Stunden bei 25°C. Man filtriert, wäscht den Rückstand mit Äther, trocknet die ätherische Lösung über Magnesiumsulfat und dampft im Vakuum ein. Nach Chromatographie des Rückstandes an Kieselgel mit Essigester / Hexan (4:1) erhält man 150 mg der Titelverbindung als Öl.

IR: 3600, 3420 (breit), 2942, 2860, 1600, 1588, 1498, 976/cm.

**Beispiel 34**

(13E)-(9S, 11R, 15R)-9-Fluor-1, 11, 15-trihydroxy-16-phenoxy-17, 18, 19, 20-tetranor-13-prosten

In Analogie zu Beispiel 33 erhält man aus 200 mg (13E)-(9S, 11R, 15R)-11, 15-Dihydroxy-9-fluor-16-phenoxy-17, 18, 19, 20-tetranor-13-prostensäuremethylester (hergestellt nach Beispiel 4) 160 mg der Titelverbindung als Öl.

IR: 3600, 3420 (breit), 2948, 2860, 1600, 1588, 1495, 975/cm.

**Beispiel 35**

(5Z, 13E)-(9R, 11R, 15R)-16, 16-Dimethyl-9-fluor-1, 11, 15-trihydroxy-5, 13-prostadien

In Analogie zu Beispiel 33 erhält man aus 100 mg (5Z, 13E)-(9R 11R, 15R)-11, 15-Dihydroxy-16, 16-dimethyl-9-fluor-5, 13-prostadien-säuremethylester (hergestellt nach Beispiel 5) 70 mg der Titelverbindung als Öl.

IR: 3600, 3410 (breit), 2952, 2858, 978/cm.

**Beispiel 36**

(13E)-(9R, 11R, 16RS)-16, 19-Dimethyl-9-fluor-1, 11, 15 α-trihydroxy-13, 18-prostadien

In Analogie zu Beispiel 33 erhält man aus 100 mg (13E)-(9R, 11R, 16RS)-11, 15α-Dihydroxy-16, 19-dimethyl-9-fluor-13, 18-prostadien-säuremethylester (hergestellt nach Beispiel 14) 72 mg der Titelverbindung als Öl.

IR: 3600, 3420 (breit), 2962, 2840, 974/cm.

**Beispiel 37**

(13E)-(9R, 11R, 15R)-11, 15-Dihydroxy-9-fluor-16-phenoxy-17, 18, 19, 20-tetranor-13-prostensäure-methylsulfonamid

Zu einer Lösung von 200 mg (13E)-(9R, 11R, 15R)-11, 15-Dihydroxy-9-fluor-16-phenoxy-17, 18, 19, 20-tetranor-13-prostensäure (hergestellt nach Beispiel 19) in 5 ml Dimethylformamid gibt man bei 0° C 90 mg Chlorameisensäureisobutylester und 70 mg Triäthylamin. Nach 30 Minuten gibt man 300 mg des Natriumsalzes von Methylsulfonanid (hergestellt aus Methylsulfonamid und Natriummethylat) und 2 ml Hexamethylphosphorsäuretriamid zu und rührt 5 Stunden bei 20° C. Anschließend verdünnt man mit Citratpuffer (pH4), extrahiert mit Essigester, wäscht den Extrakt mit Sole, trocknet über Magnesiumsulfat und dampft im Vakuum ein. Nach Chromatographie des Rückstandes an Kieselgel mit Methylenchlorid erhält man 150 mg der Titelverbindung als Öl.

IR: 3600, 3405, 2950, 2856, 1718, 1600, 1588, 978/cm.

**Beispiel 38**

(13E)-(9S, 11R, 15R)-11, 15-Dihydroxy-9-fluor-16-phenoxy-17, 18, 19, 20-tetranor-13-prostensäure-methylsulfonamid

In Analogie zu Beispiel 37 erhält man aus 100 mg (13E)-(9S, 11R, 15R)-11, 15-Dihydroxy-9-fluor-16-phenoxy-17, 18, 19, 20-tetranor -13-prostensäure (hergestellt nach Beispiel 20) 60 mg der Titelverbindung als Öl.

IR: 3600, 3410, 2950, 2856, 1720, 1600, 1588, 978/cm.

**Beispiel 39**

(13E)-(9R, 11R, 16RS)-11, 15α-Dihydroxy-16, 19-dimethyl-9-fluor-13, 18-prostadiensäure-methylsulfonanid

In Analogie zu Beispiel 37 erhält man aus 100 mg (13E)-(9R, 11R, 16RS)-11, 15 α-Dihydroxy-16, 19-dimethyl-9-fluor-13, 18-prostadiensäure (hergestellt nach Beispiel 30) 65 mg der Titelverbindung als Öl.

IR: 3600, 3420, 2958, 2852, 1720, 1480, 976/cm.

**Beispiel 40**

(13E)-(9R, 11R, 15R)-11, 15-Dihydroxy-9-fluor-16-phenoxy-17, 18, 19, 20-tetranor-13-prostensäure-isopropylsulfonamid

In Analogie zu Beispiel 37, wobei man aber an Stelle des Natriumsalzes von Methylsulfonamid das Natriumsalz von Isopropylsulfonamid verwendet, erhält man aus 150 mg (13E)-(9R, 11R, 15R)-11, 15-Dihydroxy-9-fluor-16-phenoxy-17, 18, 19, 20-tetranor-13-prostensäure (hergestellt nach Beispiel 19) 105 mg der Titelverbindung als Öl.

IR: 3600, 3410, 2955, 2848, 1717, 1600, 1588, 976/cm.

**Beispiel 41**

(13E)-(9R, 11R, 15R)-11, 15-Dihydroxy-9-fluor-16-phenoxy-17, 18, 19, 20-tetranor-13-prostensäure-acetylamid

Eine Lösung von 600 mg (13E)-(9R, 11R, 15R)-11, 15-bis-(tetrahydro-pyran-2-yloxy)-9-fluor-16-phenoxy-17, 18, 19, 20-tetranor-13-prosten-säure-methylester (hergestellt nach Beispiel 3) in 10 ml Methanol wird mit 2,5 ml 2N Natronlauge 5 Stunden unter Argon gerührt. Man engt im Vakuum ein, verdünnt mit Sole, säuert mit Citronensäure auf pH 4,5 an und extrahiert einige Male mit Essigester. Der organische Extrakt wird mit Sole gewaschen, über Magnesiumsulfat getrocknet und im Vakuum eingedanpft. Man erhält als Rückstand 560 mg (13E)-(9R, 11R, 15R)-11, 15-bis-(tetrahydropyran-2-yloxy)-9-fluor-16-phenoxy-17, 18, 19, 20-tetranor-13-prostensäure. Zur Bildung des Acetylanids löst man die Säure in 15 ml Acetonitril, versetzt mit 135 mg Triäthylamin und tropft bei 0° C eine lösung von 106 mg Acetylisocyanat in 10 ml Acetonitril zu. Nach 2 Stunden bei 20° C wird mit Citratpuffer (pH 4) verdünnt, mit Äther extrahiert, der Extrakt mit Sole gewaschen, über Magnesiumsulfat getrocknet und im Vakuum eingedampft. Zur Abspaltung der Schutzgruppen rührt man den Rückstand mit 15 ml Eisessig/Wasser/Tetrahydrofuran (65/35/10) 4 Stunden bei 40° C und dampft dann im Vakuum ein. Der Rückstand wird an Kieselgel mit Methylenchlorid unter Zusatz von 0,2-1% Isopropylalkohol chromatographiert. Man erhält 250 mg der Titelverbindung als Öl.

IR: 3600, 3410, 2952, 2858, 1712, 1600, 1588, 976/cm.

**Beispiel 42**

(13E)-(9R, 11R, 15R)-11, 15-Dihydroxy-9-fluor-16-phenoxy-17, 18, 19, 20-tetranor-13-prostensäure-amid

Zu einer Lösung von 200 mg (13E)-(9R, 11R, 15R)-11, 15-Dihydroxy-9-fluor-16-phenoxy-17, 18, 19, 20-tetranor-13-prostensäure (hergestellt nach Beispiel 19) in 5 ml Tetrahydrofuran gibt man 80 mg Chlorameisensäureisobutylester und 60 mg Triäthylamin und rührt 1 Stunde bei 0° C, leitet dann bei 0° C 15 Minuten Ammoniakgas ein und läßt 1 Stunde bei 25° C stehen. Anschließend verdünnt man mit Wasser, extrahiert mehrmals mit Methylenchlorid, wäscht die vereinigten Extrakte mit Sole, trocknet über Magnesiumsulfat und dampft im Vakuum ein. Zur Reinigung wird an Kieselgel mit Methylenchlorid (0,2-1% Isopropylalkohol chromatographiert. Man erhält 145 mg der Titelverbindung als Öl.

IR: 3600, 3505, 3410, 2955, 2848, 1670, 1605, 1590, 978/cm.

**Beispiel 43**

(5Z, 13E)-(9R, 11R, 15R)-11, 15-Dihydroxy-9-fluor-16-phenoxy-17, 18, 19, 20-tetranor-5,13-prostadiensäure-tris(hydroxymethyl)aminomethansalz

Zu einer Lösung von 200 mg (5Z, 13E)-(9R, 11R, 15R)-11, 15-Dihydroxy-9-fluor-16-phenoxy-17, 18, 19, 20-tetranor-5, 13-prostadiensäure (hergestellt nach Beispiel 17) in 35 ml Acetonitril gibt man bei 65° C eine Lösung von 60 mg Tris-(hydroxymethyl)-aminomethan in 0,2 ml Wasser. Unter Rühren läßt man auf 20° C abkühlen, dekantiert vom Lösumgsmittel und trocknet den Rückstand im Vakuum. Man erhält 180 mg der Titelverbindumg als zähes Öl.

**Patentansprüche**

1. 9-Fluor-prostaglandinderivate der allgemeinen Formel 1 worin

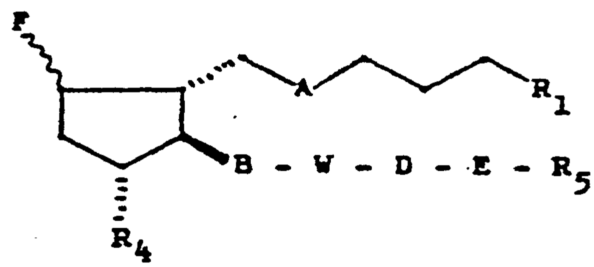

(I) ,

$R_1$ den Rest $CH_2OH$ oder

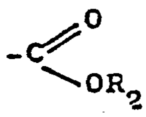

mit $R_2$ in der Bedeutung eines Wasserstoffatoms oder einer Alkylgruppe mit 1-4 C-Atomen, oder $R_1$ den Rest

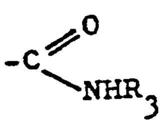

mit $R_3$ in der Bedeutung eines $C_{1-10}$-Alkanoyl- oder Methan- oder Isopropansulfonylrestes oder des Restes $R_2$ und

A eine $-CH_2-CH_2-$ oder cis-$CH=CH$-Gruppe,

B eine $-CH_2-CH_2-$, oder trans-$CH=CH$- oder eine $-C\equiv C$-Gruppe,

W eine freie oder funktionell abgewandelte Hydroxymethylengruppe, wobei die OH-Gruppe α- oder β-ständig sein kann,

D und E gemeinsam eine direkte Bindung oder

D eine gerad- oder verzweigtkettige Alkylengruppe mit 1-10 C-Atomen, die gegebenenfalls durch Fluoratome substituiert sein kann,

E ein Sauerstoff- oder Schwefelatom, eine direkte Bindung, eine $-C\equiv C$-Gruppe oder eine $-CR_6=CR_7$-Gruppe darstellt, wobei $R_6$ und $R_7$ sich unterscheiden und ein Wasserstoffatom, ein Chloratom oder eine Alkylgruppe bedeuten,

$R_4$ eine freie oder funktionell abgewandelte Hydroxygruppe,

$R_5$ ein Wasserstoffatom, eine gerad- oder verzweigtkettige, gesättigte oder ungesättigte Alkylgruppe mit 1-6 C-Atomen oder, wenn E ein Sauerstoffatom bedeutet, eine Phenylgruppe bedeuten, wobei die Gruppierung -$DER_5$ nicht den Rest n-Pentyl darstellt,

und falls $R_2$ die Bedeutung eines Wasserstoffatoms hat, deren Salze mit physiologisch verträglichen Basen.

2. Verfahren zur Herstellung von 9-Fluor-prostaglandinderivaten der allgemeinen Formel I, dadurch gekennzeichnet, daß man in an sich bekannter Weise eine Verbindung der allgemeinen

Formel II

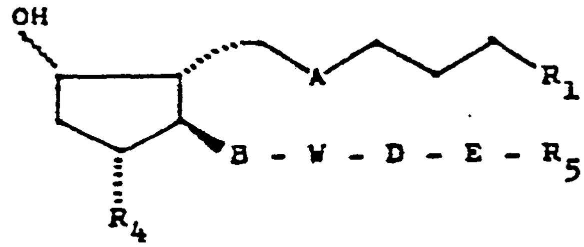

(II),

worin die OH-Gruppen α- oder β-ständig sein können, $R_1$ die Bedeutung der Reste

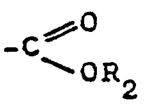

oder

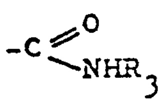

aufweist und A, B, D, E und $R_5$ die oben angegebenen Bedeutung haben und freie OH-Gruppen in $R_4$ und W geschützt sind, über einen intermediären Sulfonsäureester mit einem Tetraalkylammoniumfluorid umsetzt und gegebenenfalls anschließend in beliebiger Reihenfolge geschützte Hydroxygruppen freisetzt und/oder freie Hydroxygruppen verestert oder veräthert und/oder Doppelbindungen hydriert, und/oder eine veresterte Carboxylgruppe

($R_1$ = 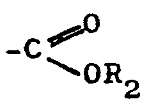 )

verseift und/oder eine Carboxylgruppe ($R_2$ = H) verestert und/oder eine freie Carboxylgruppe ($R_2$ = H) in ein Amid

($R_1$ = 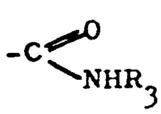 )

oder Salz Überführt und/oder eine freie oder veresterte Carboxylgruppe

($R_1$ = 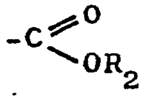 )

reduziert und/oder falls $R_2$ die Bedeutung eines Wasserstoffatoms hat, die Überführung der Carboxylgruppe in Salze mit physiologisch verträglichen Basen.

3) Arzneimittel, bestehend aus einer oder mehreren Verbindungen gemäß Anspruch 1 und üblichen Hilfs- und

Trägerstoffen,

4) (13E)-(9R, 11R, 15R)-11, 15-Dihydroxy-9-fluor-16-phenoxy-17, 18, 19, 20-tetranor-13-prostensäure

5) (13E)-(9S, 11R, 15R)-11,15-Dihydroxy-9-fluor-16-phenoxy-17, 18, 19, 20-tetranor-13-prostensäure

6) (13E)-(9R, 11R, 15R)-11, 15-Dihydroxy-16, 16-dimethyl-9-fluor-13-prostensäure

7) (5Z, 13E)-(9S, 11R, 16RS)-11, 15 α-Dihydroxy-9-fluor-16-methyl-5, 13-prostadiensäure

8) (13E)-(9R, 11R, 16RS)-11, 15 α-Dihydroxy-16, 19-dimethyl-9-fluor-13, 18-prostadiensäure

9) (13E)-(9R, 11R, 15R)-9-Fluor-1, 11, 15-trihydroxy-16-phenoxy-17, 18, 19, 20-tetranor-13-prosten

10) (5Z, 13E)-(9R, 11R, 15R)-11, 15-Dihydroxy-9-fluor-16-phenoxy-17, 18, 19, 20-tetranor-5, 13-prostadiensäure-tris(hydroxymethyl)aminomethansalz

11) (5Z, 13E)-(9R, 11R, 15R)-11, 15-Dihydroxy-9-fluor-16-phenoxy-17, 18, 19, 20-tetranor-5, 13-prostadiensäure.

**Claims**

1. 9-fluoroprostaglandin derivatives of the general formula I,

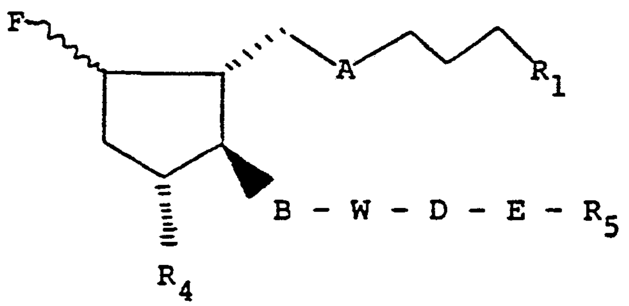

(I)

in which

$R_1$ represents the radical $CH_2OH$ or

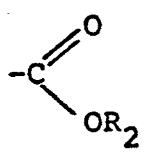

in which $R_2$ denotes a hydrogen atom or an alkyl group having from 1 to 4 carbon atoms, or $R_1$ represents the radical

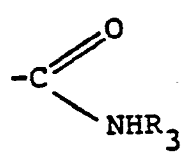

in which $R_3$ denotes a $C_{1-10}$-alkanoyl radical or a methane- or isopropane-sulphonyl radical or the radical $R_2$, and

A represents a $-CH_2-CH_2-$ or cis-$CH=CH$- group,

B represents a $-CH_2CH_2-$ group or a trans-$CH=CH$- or a $-C \equiv C-$ group,

W represents a free or functionally modified hydroxymethylene group,it being possible for the OH group to be in the α- or the β-configuration,

D and E together represent a direct bond or

D represents a straight-chain or branched-chain alkylene group having from 1 to 10 carbon atoms which may be optionally substituted by fluorine atoms, and

E represents an oxygen or sulphur atom, a direct bond, a $-C \equiv C-$ group or a $-CR_6=CR_7-$ group, in which $R_6$ and $R_7$ are different from one another and represent a hydrogen atom, a chlorine atom or an alkyl group,

$R_4$ represents a free or functionally modified hydroxy group,

$R_5$ represents a hydrogen atom, a straight-chain or branched-chain, saturated or unsaturated alkyl group

having from 1 to 6 carbon atoms or, if E represents an oxygen atom, represents a phenyl group,

and in which the grouping -$DER_5$ does not represent the radical n-pentyl,

and, if $R_2$ denotes a hydrogen atom, the salts thereof with physiologically acceptable bases.

2. Process for the manufacture of 9-fluoroprosta-glandin derivatives of the general formula I, characterised in that in a manner known per se a compound of the general formula II

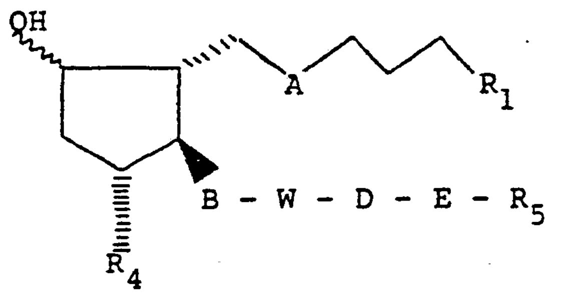

(II),

in which the OH groups may be in the α- or the βconfiguration, $R_1$ denotes the radicals

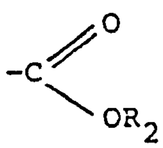

or

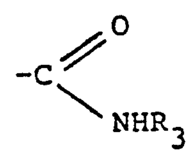

and A, B, D, E and $R_5$ have the meanings given above and free OH groups in $R_4$ and W are protected, is reacted by way of an intermediate sulphonic acid ester with a tetraalkylammonium fluoride and then, optionally, in any desired order, protected hydroxy groups are freed and/or free hydroxy groups are esterified or etherified, and/or double bonds are hydrogenated and/or an esterified carboxy group

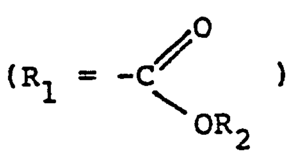

is hydrolysed and/or a carboxy group ($R_2$ = H) is esterified and/or a free carboxy group ($R_2$ = H) is converted into an amide

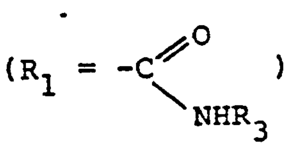

or a salt and/or a free or esterified carboxy group

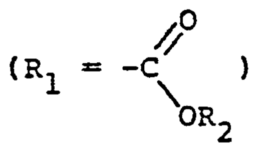

is reduced and/or if $R_2$ denotes a hydrogen atom, the carboxy group is converted into salts with physiologically acceptable bases.

3. Medicaments consisting of one or more compounds according to claim 1 and customary auxiliaries and carriers.

4. (13E)-(9R, 11R, 15R)-11, 15-dihydroxy-9-fluoro-16-phenoxy-17, 18, 19, 20-tetranor-13-prostenoic acid.

5. (13E)-(9S, 11R, 15R)-11, 15-dihydroxy-9-fluoro-16-phenoxy-17, 18, 19, 20-tetranor-13-prostenoic acid.

6. (13E)-(9R, 11R, 15R)-11, 15-dihydroxy-16, 16-dimethyl-9-fluoro-13-prostenoic acid.

7. (5Z, 13E)-(9S, 11R, 16RS)-11, 15α-dihydroxy-9-fluoro-16-methyl-5, 13-prostadienoic acid.

8. (13E)-(9R, 11R, 16RS)-11, 15α-dihydroxy-16, 19-dimethyl-9-fluoro-13, 18-prostadienoic acid.

9. (13E)-(9R, 11R, 15R)-9-fluoro-1, 11, 15-trihydroxy-16-phenoxy-17, 18, 19, 20-tetranor-13-prostene.

10. The tris(hydroxymethyl)aminomethane salt of (5Z, 13E)-(9R, 11R, 15R)-11, 15-dihydroxy-9-fluoro-16-phenoxy-17, 18, 19, 20-tetranor-5, 13-prostadienoic acid.

11. (5Z, 13E)-(9R, 11R, 15R)-11, 15-dihydroxy-9-fluoro-16-phenoxy-17, 18, 19, 20-tetranor-5, 13-prostadienoic acid.

**Revendications**

1. Fluoro-9 prostaglandines répondant à la formule générale I:

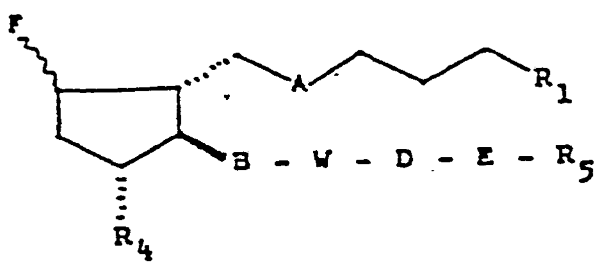

(I)

dans laquelle

$R_1$ représente un radical $-CH_2OH$ ou un radical

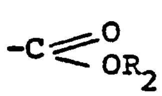

dont

le symbole $R_2$ désigne un atome d'hydrogène ou un radical alkyle contenant de 1 à 4 atomes de carbone, ou encore $R_1$ représente un radical

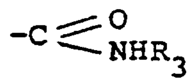

dont le symbole $R_3$ désigne un radical alcanoyle en $C_1$-$C_{10}$, un radical méthanesulfonyle, un radical isopropanesulfonyle ou un radical ayant la signification de $R_2$,

A représente un radical $-CH_2-CH_2-$ ou un radical $-CH=CH-$ cis,

B représente un radical $-CH_2-CH_2-$, un radical $-CH=CH-$ trans ou un radical $-C\equiv C-$,

W représente un radical hydroxyméthylène libre ou sous la forme d'un dérive fonctionnel, le radical -OH pouvant avoir la configuration α ou la configuration β,

D et E forment ensemble une liaison directe, ou D représente un radical alkylènelinéaire ou ramifié qui contient de 1 à 10 atomes de carbone, éventuellement porteur d'atomes de fluor, et E représente un atome d'oxygène ou de soufre, une liaison directe, un radical $-C\equiv C-$ ou un radical $-CR_6=CR_7-$, les symboles $R_6$ et $R_7$ n'ayant pas la même signification et représentant chacun un atome d'hydrogène, un atome de chlore ou un radical alkyle,

$R_4$ représente un radical hydroxy libre ou sous la forme d'un dérivé fonctionnel

et

$R_5$ représente un atome d'hydrogène, un radical alkyle linéaire ou ramifié, saturé ou insaturé, qui contient de 1 à 6 atomes de carbone, ou encore représente un radical phényle dans le cas où E désigne un atome d'oxygène, avec la condition que le groupement $-DER_5$ ne représente pas un radical n-pentyle.

et également, dans le cas où $R_2$ représente un atome d'hydrogène, les sels que forment ces composés avec des bases acceptables du point de vue physiologique.

2. Procédé pour préparer des fluoro-9 prostaglandines de formule générale I, procédé caractérisé en ce qu'on fait réagir, demanière connue, un composé repondant à la formule générale II:

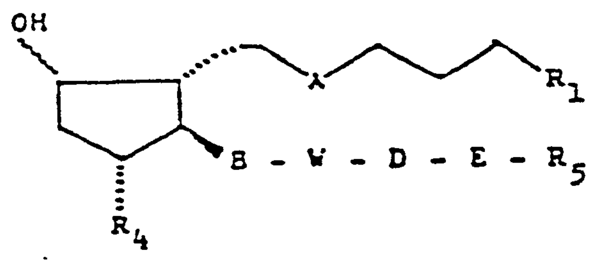

(II),

(dans laquelle les radicaux -OH peuvent avoir la configuration α ou la configuration β, $R_1$ représente un radical

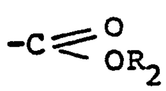

ou un radical

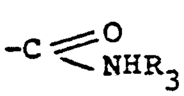

A, B, D, E, et $R_5$ ont les significations indiquées plus haut et les radicaux -OH dans $R_4$ et W sont protégés), en passant par un ester sulfonique intermédiaire, avec un fluorure de tétra-alkylammonium, après quoi, en opérant dans l'ordre que l'on veut, on effectue éventuellement les réactions suivantes:

on libère des radicaux hydroxy protégés et/ou on estérifie ou éthérifie des radicaux hydroxy libres et/ou on hydrogène des doubles liaisons et/ou on saponifie un radical carboxy estérifié

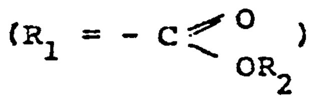

et/ou on estérifie un radical carboxy ($R_2$= H)
et/ou on transforme un radical carboxy libre ($R_2$=H) en un radical d'amide

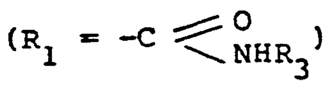

ou en un sel
et/ou on réduit un radical carboxy libre ou estérifié

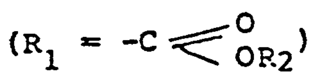

et/ou, dans le cas où $R_2$ désigne un atome d'hydrogène, on effectue la transformation du radical carboxy en un radical de sel avec une base acceptable du point de vue physiologique.

3. Médicament constitué d'un ou plusieurs composés selon la revendication 1 et de supports et excipients usuels.

4. Acide dihydroxy-11, 15 fluoro-9 phénoxy-16 tétranor-17, 16, 19, 20 prostène-13 oïque-(13E)-(9R, 11R, 15R).

5. Acide dihydroxy-11, 15 fluoro-9 phénoxy-16 tétranor-17, 16, 19, 20 prostène-13 oïque-(13E)-(9S, 11R, 15R).

6. Acide dihydroxy-11, 15 diméthyl-16, 16 fluoro-9 prostène-13 oïque (13E) (9R, 11R, 5R).

7. Acide dihydroxy-11, 15 α fluoro-9 méthyl-16 prostadiène-5, 13 oïque-(5Z, 13E)-(9S, 11R, 16RS).

6. Acide dihydroxy-11, 15 α diméthyl-16, 19 fluoro-9 prostadiène-13, 18 oïque-(13E)-(9R, 11R, 16RS).

9. Fluoro-9 trihydroxy-1, 11, 15 phénoxy-16 tétranor-17, 18, 19, 20 prostène-13 (13E)-(9R, 11R, 15R).

10. Sel formé par l'acide dihydroxy-11, 15 fluoro-9 phénoxy-16 tétranor-17, 18, 19, 20 prostadiène-5, 13 oïque-(5Z, 13E) -(9R, 11R, 15R) et le tris-(hydroxyméthyl)-amino-méthane.

11. Acide dihydroxy-11, 15 fluoro-9 phénoxy-16 tétra-nor-17, 16, 19, 20 prostadiène-5, 13 oïque-(5Z, 13E)-(9R, 11R, 15R).